# EUROPEAN PATENT SPECIFICATION

(11) **EP 1 237 534 B1**
(45) Date of publication and mention of the grant of the patent: **19.01.2005**
(21) Application number: 00979577.4
(22) Date of filing: 13.11.2000
(51) Int. Cl.: A61K 7/48, A61K 7/50, C11D 1/32

(54) **STABLE, HIGH GLYCEROL LIQUIDS COMPRISING SULFOSUCCINIC ACID MONOESTERS**
STABILE FLÜSSIGKEITEN MIT HOHEM GLYCEROLGEHALT, DIE SULFOSUCCINSÄUREMONOESTER ENTHALTEN
LIQUIDES STABLES A FORTE TENEUR EN GLYCEROL CONTENANT DES MONOESTERS D'ACIDE SULFOSUCCINIQUE

(30) Priority: 06.12.1999 GB 9928820
(43) Date of publication of application: 11.09.2002
(73) Proprietor: UNILEVER PLC, London EC4P 4BQ (GB); UNILEVER N.V., 3013 AL Rotterdam (NL)
(72) Inventor: ARAI, Masaaki, Unilever Home & Personal Care USA, Trumbull, CT 06611 (US); KOBAYASHI, Mitsunobu, Nippon Lever B.V., Tochigi, Tochigi 321-3325 (JP); MISHINA, Yukie, Obihiro-shi, Hokkaido 080-0044 (JP); TAKADA, Naohiko, Nippon Lever B.V., Tochigi, Tochigi 321-3325 (JP)
(74) Representative: Mulder, Cornelis Willem Reinier, Dr.
(86) International application number: PCT/EP2000/011359
(87) International publication number: WO 2001/041729

(56) References cited:
- EP-A- 0 995 433
- WO-A-00/06677
- WO-A-99/29409
- JP-A- 10 237 494
- US-A- 4 284 534
- US-A- 5 130 056
- US-A- 5 132 037
- US-A- 5 939 378
- DATABASE WPI Section Ch, Week 199109 Derwent Publications Ltd., London, GB; Class A21, AN 1991-060270 XP002163989 & HU 54 191 A (OFFSET ES JATEKKART), 28 January 1991 (1991-01-28)

## Description

### Field of the Invention

The present invention relates to high glycerin content liquid compositions comprising one or more defined sulfosuccinic acid monoesters. More specifically, while high glycerin content has been found to destabilize such compositions, applicants have found that a combination of a minimum level of fatty acid salt (e.g., alkali metal salt or alkanolamine salt), and a thickener selected from acrylate copolymers, hydroxy alkyl cellulose, cationic guar gums (e.g., Jaguar C13S) stabilize the compositions. The compositions of the invention may be used in skin cleansing, shower gel, and hair compositions.

US 5,130,056 to Jakobson *et al*. relates to a cleaning composition, and a process for preparing a composition containing at least one anionic surfactant consisting of a fatty acid ester and at least one ionic and/or amphoteric surfactant.

US 5,132,037 to Greene *et al*. describes cleansers containing long chain acylisethionate surfactants, free isethionate salts, long chain fatty acids and moisturising agents. The cleansers disclosed in US 5,132,037 have good foaming and moisturising properties yet are nto harsh on the skin.

US 4,284,534 to Ehrlich *et al*. relates to a bubble blowing apparatus and bubble composition which is non-toxic and does not irritate the eyes. The composition includes lauric diethanolamide, an alkanolamido half ester of a sulfosuccinic acid salt, a water soluble film-forming agent, glycerine and water.

WO 99/29409 in the name of Betzdearbom Inc, discloses a composition for inhibiting microbial adhesion on surfaces which is water, oil and alcohol feee yet contains a stable solution of dialkylsulfosuccinate and a non-ionic ethylene oxide/propylene oxide block polymer.

Compositions containing sulfosuccinic acid monoesters are disclosed in U.S. Patent No. 4,749,515 to Miyamoto *et al*..

However, because of its moisturizing properties, it would also be greatly beneficial to add glycerin to such compositions to relieve skin dryness. In such compositions, however, it has been found that large amounts of glycerin (e.g., 10% by wt. and above) lead to instability and phase separation. While not wishing to be bound by theory, it is thought that this may be due to the high specific gravity of glycerin.

Unexpectedly, applicants have now found that this instability may be overcome by using a stabilization system comprising:
(a) at least 4% by wt. of an alkali metal soap of C₁₂-C₂₄ fatty acid and/or alkanolamine soap; and
(b) 0.01 to 5% by wt. of a thickening copolymer selected from acrylate copolymers, hydroxy alkyl cellulose (e.g., hydroxy ethyl or propyl cellulose), cationic guar gum, and isostearic acid. Acrylate copolymers are particularly preferred.

### Brief Summary of the Invention

The present invention relates to stable, high glycerin liquid detergent compositions. More particularly, the compositions comprise:
(1) 1-25%, preferably 5 to 20% of one or more sulfosuccinic acid monoesters represented by Formula (I), (II), (III) or (IV); 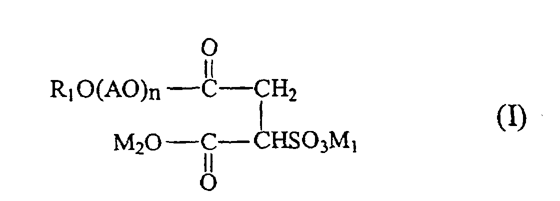 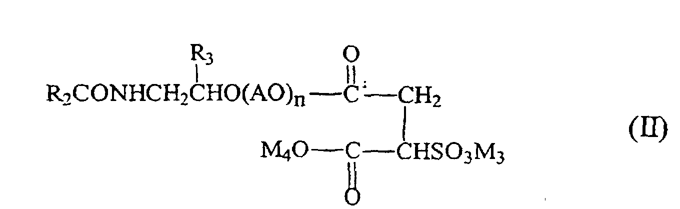 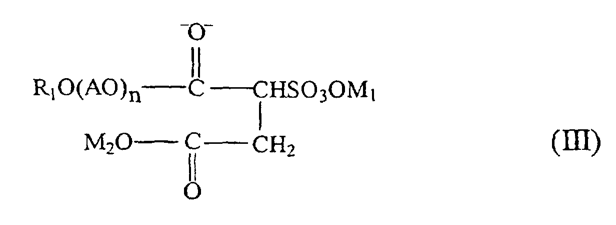 or 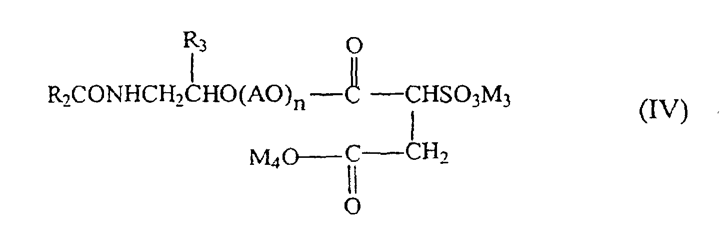 wherein each of M₁ to M₄ is H, NH₄, an alkali metal or a hydroxyalkyl substituted ammonium. R₁ and R₂ are each an alkyl or hydroxyalkyl group having about 8 to 20 carbon atoms, R₃ is H or CH₃, AO is an oxyalkylene group having 2 or 3 carbon atoms, and n represents an integer from 0 to 20;
(2) 10% and above, preferably 10 to 50% by wt., preferably 15 to 40% by wt. of glycerin;
(3) at least 4% by wt., preferably at least 5% to 15% by wt. of an alkali metal soap of C₁₂-C₂₄ fatty acid and/or alkanolamine soap;
(4) 0.01 to 5% by wt., preferably 0.1 to 3% by wt. (levels may vary depending on which thickener is used) of a thickener selected from acrylate copolymer (e.g., alkyl acrylate copolymer), hydroxy alkyl cellulose, cationic guar gum and isostearic acid; and
(5) balance water.

In a second embodiment, the invention relates to a method of enhancing the stability of high glycerin compositions comprising defined sulfosuccinic acid monoesters by adding a stabilization system comprising components (3) and (4) above.

The pH of the compositions is about 6 to 8.5, preferably 6.5 to 8.5 and viscosity is about 60 to 3000 cm²/minute (100 to 5000 centistokes) preferably 60 to 120 cm²/minute (100 to 200) measured using a Brookfield Viscometer, Spindle No.2 or 3, 12 rpm, for about 30 seconds at 25°C.

The stability of the composition is defined by the absence of separation after 4 weeks storage at room temperature.

### Detailed Description of the Invention

The present invention relates to liquid compositions comprising one or more defined sulfosuccinic acid monoesters and relatively high amounts of glycerin while retaining stability. More specifically, by using a combination of fatty acid salts and specific thickeners (preferably acrylate copolymers and isostearic acid), it has been found that stable, high content glycerin (greater than or equal to 10% by wt. to about 50% by wt. glycerin), sulfosuccinic monoester compositions may be formed.

Compositions of the present invention are set forth in greater detail below.

The compositions of the present invention comprise 1-25%, preferably 5-20% by wt. of one or more sulfosuccinic acid monoesters represented by Formula I, II, III or IV and mixtures thereof as follows: 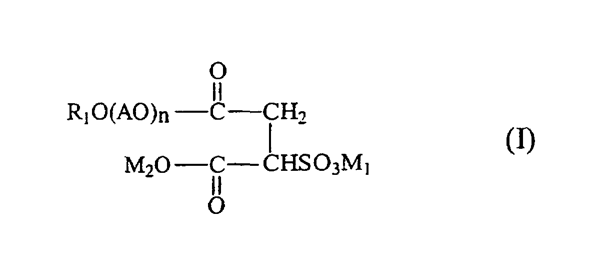 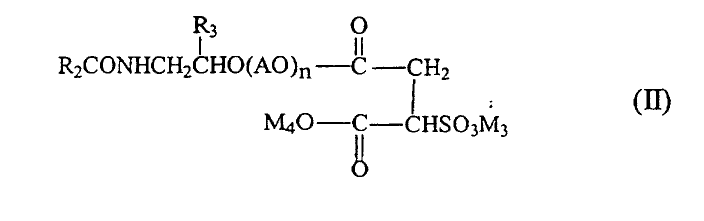 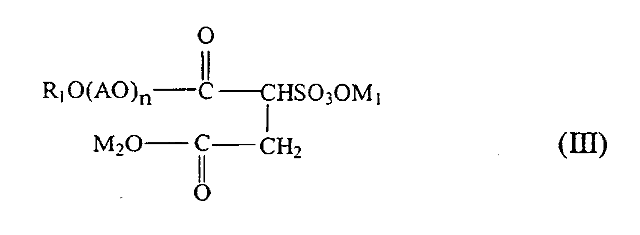 or

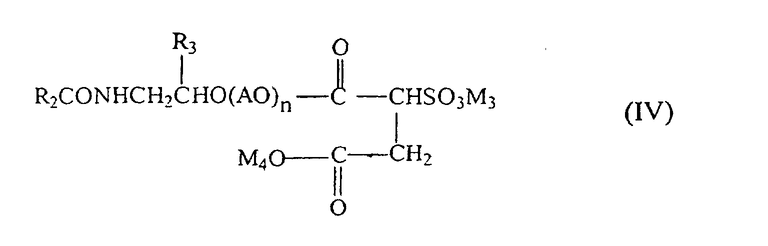

In these formulae, each of M₁ to M₄ may be selected from H, NH₄, an alkali metal and hydroxyalkyl-substituted ammonium groups. M₁ and M₂, and M₃ and M₄ may be the same or different. Alkali metals may include lithium, sodium, potassium and the like. The hydroxyalkyl substituted ammonium groups, which may preferably have 1 to 3 carbon atoms in the hydroxyalkyl group, may include monoethanol ammonium, diethanol ammonium, triethanol ammonium, methyl diethanol ammonium and the like. Preferably, M₁ to M₄ are hydrogen, sodium or triethanol ammonium.

R₁ and R₂ are each straight or branched alkyl or hydroxyalkyl groups having about 8 to 20 carbon atoms, for example, hexyl, decyl, hydroxydecyl, dodecyl, hydroxytetradecyl, tetradecyl, nonadecyl, or the like. Any alkyl group having less than 8 or more than 20 carbon atoms may optionally be contained in the molecule, provided that the total number of carbon atoms in the sulfosuccinic acid monoester contained in the detergent composition of the present invention is in the range from 8 to 20. This number of carbon atoms provides good foaming properties, whereas when the number of carbon atoms is either less than 8 or more than 20 less foaming is observed.

In the formulae (II) and (IV), R₃ is either H or CH₃.

In the formulae, AO represents an oxyalkylene group having 2 or 3 carbon atoms, that is, oxyethylene or oxypropylene group. Both oxyethylene and oxypropylene groups may be present in the molecule.

The letter n represents an integer from 0 to 20, preferably 1 to 10. If n is more than 20, the foaming properties of the resulting liquid detergent compositions may be poor.

The compounds represented by the formulae (I), (II), (III) and (IV) may be prepared by any known methods.

For example, the sulfosuccinic acid monoesters represented by the general formulae (I) or (II) may be prepared by reacting an alkylene oxide adduct of a higher fatty acid with maleic anhydride to produce an ester of maleic anhydride and further reacting the ester of maleic anhydride with a sulfite.

Alternatively, the sulfosuccinic acid monoesters represented by the general formulae (II) or (IV) may be prepared by reacting a lower alcohol ester of a higher fatty acid with an alkanolamine, adding an alkylene oxide to the reaction product, further reacting the resulting addition product with maleic anhydride to produce an ester of maleic anhydride, and reacting the ester of maleic anhydride with a sulfite.

As part of the required stability system of the invention, compositions of the invention also require at least 4%, preferably at least 5% to about 15% by wt. of an alkali metal soap of a C₁₂-C₁₄ fatty and/or alkanolamine salt.

Suitable fatty acid soaps include, but are not limited to, sodium or potassium salts of lauric or myristic acid. Suitable alkanolamine salts include but are not limited to ethanolamine, triethanolamine etc.

A second component of the stability system is a thickener. The thickener is selected from acrylate copolymers, hydroxy alkyl celluloses, cationic guar gums, and isostearic acid.

Among the acrylates which may be used are included alkyl acrylate copolymer e.g. Leoarl MS200 ex Lion and Acuryn 33 ex Rohm and Haas; Acryl copolymer e.g. Auryn 22 ex Rohm and Haas. Hydroxy alkyl celluloses include hydroxy ethyl, hydroxy propyl or hydroxy methyl cellulose. Cationic guar gums include Jaguar gums.

The thickener may be used in amounts between 0.01 to 5% by wt. although the amounts may depend on which thickener is used. For example, acrylate copolymer may comprise 0.1-3% by wt. while isostearic acid may comprise from 1-5% by wt of the composition.

The balance of the composition (generally about 20-70%, preferably 25-65% by wt.) is water.

### Optional Components

Compositions of the invention may contain 0.1 to 10% by wt. of a surfactant selected from anionic, nonionic, amphoteric and cationic surfactants and mixtures thereof.

Suitable anionic detergents include, but are not limited, to alkyl and alkylene carboxylates having 10 to 20 carbon atoms, alkyl ether carboxylates, fatty alcohol sulfates, fatty alcohol ether sulfates, alkylol amido-sulfates and sulfonates, fatty acid alkylol amido-polyglycol ether sulfates, alkane sulfonates and hydroxyalkane sulfonates, olefin sulfonates, acyl esters of isethionates, α-sulfo fatty acid esters, alkyl-benzene sulfonates, alkylphenol glycol ether sulfonates, sulfo-succinates, sulfosuccinic acid half and di-esters, fatty alcohol ether phosphates, protein fatty acid condensation products, alkyl monoglyceride sulfates and sulfonates, alkyl glyceride ether sulfonates, fatty acid methyl taurides, fatty acid sarcosinates, sulfo-ricinoleates. These compounds and mixtures thereof are used in the form of water-soluble or water-dispersible salts, for example sodium, potassium, magnesium, ammonium, mono-, di- and triethanol-ammonium salts and analogous alkylol ammonium salts.

A particularly preferred anionic surfactant is polyoxyalkylene alkylether sulfate which may be used in an amount of, for example of 0.01 to 5% by wt.

Suitable nonionic surface-active agents include,for example fatty alcohol ethoxylates (alkyl-polyethylene glycols), alkylphenol-polyethylene glycols, alkylation-polyethylene glycols, fatty amine ethoxylates (alkylamino-polyethylene glycols), fatty acid ethoxylates (acyl polyethylene glycols), polypropylene glycol ethoxylates (Pluronic (Registered Trademark)), fatty acid alkyl amides (fatty acid amino polyethylene glycols), saccharose esters, sorbitol esters, and polyglycol ether.

Suitable amphoteric surface-active agents to be added to the shampoos include N-alkyl-β-amino dipropionates having 12 to 18 alkyl carbon atoms as alkali metal salts and mono-, di-and trialkylol-ammonium salts, N-acylamido-alkyl-N, N-dimethylacetobetaine, preferably N-acyl-amidopropyl-N,N-dimethylacetobetaine having 8 to 18 acyl carbon atoms, alkyl-dimethylsulfopropyl-betaine having 12 to 18 alkyl carbon atoms, amphoteric surfactants of the imidazoline type (Trademarks: Miranol Steinapon), preferably the sodium salt of 1-(β-carboxy-methyloxethyl)-1-(carboxymethyl)-2-lauryl-imidazolinium; amine oxides, for example alkyl-dimethyl-amine oxide having 12 to 18 alkyl carbon atoms, fatty acid amidoalkyl-dimethylamine oxide.

A particularly preferred amphoteric surfactant is cocoamide propyl betaine.

Among suitable cationics are, for example, quaternary ammonium salts, such as dialkyl-dimethyl-ammonium, chloride or bromide having 10 to 24, preferably 12 to 18 carbon atoms, in the alkyl portion, alkyl-dimethyl-ethylammonium chloride or bromide having 10 to 24 alkyl carbon atoms, alkyl-trimethyl-ammonium chloride or bromide having 10 to 24 alkyl carbon atoms, preferably cetyl-trimethyl ammonium chloride or bromide, alkyl-trimethyl ammonium chloride or bromide having 10 to 22 alkyl carbon atoms, or alkyl-dimethyl-benzyl ammonium chloride or bromide having 10 to 24, preferably 12 to 18, carbon atoms in the alkyl portion.

### Other optional components are as follows:

The liquid personal cleansing compositions of the present invention may optionally also include water-dispersible, gel-forming polymers. This polymer is preferably an anionic, nonionic, cationic or hydrophobically modified polymer, selected from cationic polysaccharides of the cationic guar gum class with molecular weights of 1,000 to 3,000,000, anionic, cationic and nonionic homopolymers derived from acrylic and/or methacrylic acid, anionic, cationic and nonionic cellulose resins; cationic copolymers of dimethyldialkyl ammonium chloride and acrylic acid; cationic homopolymers of dimethyldialkyl ammonium chloride; cationic polyalkylene and ethoxypolyalkylene imines polyethylene glycol having molecular weights from 10,000 to 4,000,000; and mixtures thereof. Preferably, the polymer is selected from Sodium Polyacrylate, Hydroxy Ethyl Cellulose, Cetyl Hydroxy Ethyl Cellulose, and Polyquaternium 10.

The polymer is preferably included in the compositions of the present invention at a level of from about 0.1 parts to 1 part, more preferably 0.1 parts to 0.5 parts of the composition. The polymers improve the sensory feel of the lipid on skin in addition to providing product stabilization. The improved sensory feel results from reduced tackiness and greasiness and improved smoothness. In an especially preferred embodiment a mixture of polymers is used, for example those polymers preferred for product stabilization, and/or those preferred for improved sensory feel. Preferred polymers for improved sensory feel are selected from polyethylene glycol, hydroxypropyl guar, guar hydroxypropyl trimonium chloride, polyquaternary 3, 5, 6, 7, 10, 11 and 24 and mixtures thereof.

A variety of additional ingredients may be incorporated into the compositions of the present invention. These materials include, but not limited to, liquid appearance aids, salts and their hydrates and other "filler materials" as listed in U.S. Patent No. 5,340,492 to Kacher *et al*., and U.S. Patent No, 4,919,934 to Deckner *et al*.

Other non-limiting examples of these additional ingredients include vitamins and derivatives thereof (e.g., ascorbic acid, vitamin E, tocopherol acetate, and the like); sunscreens; thickening agents (e.g., polyol alkoxy ester, available as Crothix from Croda at levels up to 2% and xanthan gum at levels up to 2%); preservatives for maintaining the anti-microbial integrity of the compositions; anti-acne medicaments (resorcinol, salicylic acid, and the like); antioxidants; skin soothing and healing agents such as aloe vera extract, allantoin and the like; chelators (e.g., EDTA and hydroxy ethan diphosphoric acid) and sequestrants; and agents suitable for aesthetic purposes such as fragrances, essential oils, skin sensates, pigments, pearlescent agents (e.g., mica and titanium dioxide), additives to impart a draggy rinse feel (e.g., fumed silica), additives to enhance deposition (e.g., maleated soybean oil at levels up to 3%), lakes, colorings, and the like (e.g., clove oil, menthol, camphor, eucalyptus oil and eugenol).

The compositions of the present invention have a pH of about 6 to 8.5, preferably 6.5 to 8.5.

Compositions of the present invention have a viscosity of 60 - 3000 cm²/minute (100-5000 centistokes) measured using a Brookfield Viscometer, Spindle 2 and 3 at 12 rpm for 30 secohds at 25°C.

The compositions of the present invention exhibit no precipitation or phase separation after 4 weeks of storage at room temperature (i.e., about 25°C).

### EXAMPLES

### Examples 1, 2 and 3

The following examples were prepared as follows:

| | **Example1** | **Example2** | **Example3** |
|---|---|---|---|
| Disodium Laurylether sulfsuccinate(DSLES) | 4.25% | 5% | 7% |
| Disodium Lauryl sulfsuccinate(DSLS) | 4.25% | 5% | 7% |
| Potassium Laurate | | 3% | 3% |
| Potassium Myristate | | 2% | 2% |
| Lauric acid | 4.25% | | |
| Isostearic acid | | | 4% |
| Potassium hydroxyde | 0.6% | | |
| Cocamido propyl betaine(CAPB) | 2.6% | 3% | 0.6% |
| Sodium Laurylether sulfate(SLES) | 1.7% | 2% | 0.4% |
| Glycerine | 20% | 20% | 20% |
| Dipropylene glycol | | 3% | |
| Amphoteric polymer | 0.3% | | 0.3% |
| Cationic polymer | | 0.6% | |
| Alkyl acrylate polymer | 0.5% | 0.5% | 0.5% |
| Ethylene glycol distearate(EGDS) | 2% | 2% | 2% |
| Dibutylhydoxy toluene(BHT) | 0.05% | 0.05% | 0.05% |
| EDTA | 0.05% | 0.05% | 0.05% |
| Hydroxy ethane diphosphoric acid | | 0.2% | 0.2% |
| Preservative | 0.3% | 0.3% | 0.3% |
| Perfume | 0.7% | 0.7% | 0.7% |
| Water | To 100% | To 100% | To 100% |

### Processing: Example 1

Example 1 above was processed as follows:
1) A mixture of glycerin, water, potassium hydroxide and SLES was heated to 75-80°C until becoming quite fluid (mixture-1);
2) Lauric acid, preservative and BHT were added to mixture-1 with agitation(mixture-2);
3) DSLES, DSLS and CAPB were added to mixture-2 with agitation(mixture-3);
4) A mixture of EDTA and water was mixed at 75-80°C (mixture-4);
5) A mixture of amphoteric polymer and water was added into mixture-4 with agitation (mixture-5);
6) EGDS and alkyl acrylate polymer were separately added to mixture-5 with agitation(mixture-6);
7) Mixture-6 was cooled to 45°C;
8) Perfume was added to mixture-9 and cooled to 35°C.

### Examples 5,6 & Comparative Example A

| | **Example 5** | **Comparative A** | **Example 6** | **Comparative B** |
|---|---|---|---|---|
| Disodium Laurylether sulfsuccinate(DSLES) | 7% | 7% | 4.25% | 4.25% |
| Disodium Lauryl sulfsuccinate(DSLS) | 7% | 7% | 4.25% | 4.25% |
| Potassium Laurate | 3% | 3% | | |
| Potassium Myristate | 2% | 2% | | |
| Lauric acid | | | 4.25% | 4.25% |
| Isostearic acid | 4% | | | |
| Potassium hydroxyde | | | 0.6% | 0.6% |
| Cocamido propyl betaine(CAPB) | 0.6% | 0.6% | 2.6% | 2.6% |
| Sodium Laurylether sulfate(SLES) | 0.4% | 0.4% | 1.7% | 1.7% |
| Glycerine | 20% | 20% | 20% | 20% |
| Dipropylene glycol | | | | |
| Amphoteric polymer | 0.3% | 0.3% | 0.3% | 0.3% |
| Cationic polymer | | | | |
| Alkyl acrylate polymer | 0.5% | 0.5% | 0.5% | |
| Ethylene glycol distearate(EGDS) | 2% | 2% | 2% | 2% |
| Dibutylhydoxy toluene(BHT) | 0.05% | 0.05% | 0.05% | 0.05% |
| EDTA | 0.05% | 0.05% | 0.05% | 0.05% |
| Hydroxy ethane diphosphoric acid | 0.2% | 0.2% | | |
| Preservative | 0.3% | 0.3% | 0.3% | 0.3% |
| Perfume | 0.7% | 0.7% | 0.7% | 0.7% |
| Water | To 100% | To 100% | To 100% | To 100% |
| RT stability: Separation | good | no good | good | no good |

From the data obtained for Example 5 and Comparative A, it can be seen that 4% isostearate provided good stability (no phase separation) while an absence of isostearic acid (Comparative A) resulted in instability. Similarly, the use of alkyl acrylate (Example 6) resulted in stability while the absence (Comparative B) resulted in instability.

## Claims

1. A stable, high glycerol liquid detergent composition comprising:
(a) 1-25%, by wt. of one or more sulfosuccinic acid monoesters represented by Formulae (I), (II), (III) or (IV). 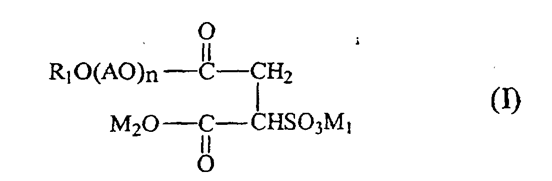 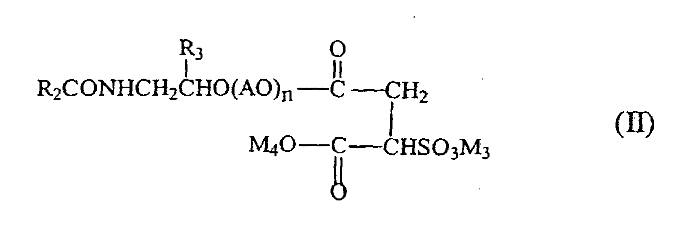 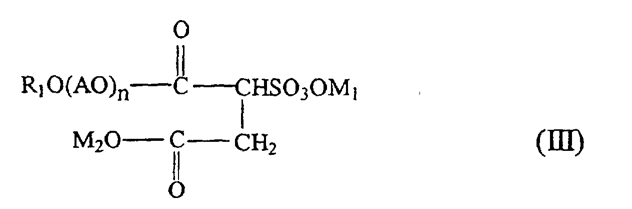 or 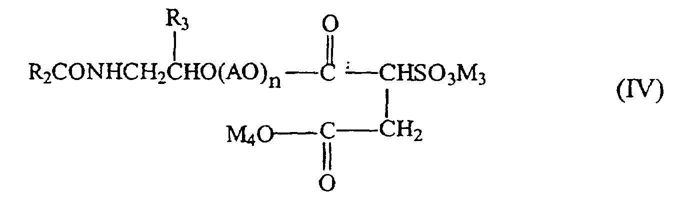 wherein each of M₁ to M₄ is H, NH₄, an alkali metal or a hydroxyalkyl substituted ammonium; R₁ and R₂ are each an alkyl or hydroxyalkyl group having about 8 to 20 carbon atoms; R₃ is H or CH₃; AO is an oxyalkylene group having 2 or 3 carbon atoms, and n represents an integer of 0 to 20;
(b) 10% to 50% by wt. glycerin;
(c) at least 4% by wt. of an alkali metal soap of C₁₂-C₂₄ fatty acid and/or alkanol soap; and
(d) 0.01-5%, by wt. of a thickener selected from acrylate copolymer, hydroxy alkyl cellulose, cationic guar gums and isostearic acid; and
(e) balance water.

2. A composition according to claim 1, wherein the composition additionally comprises 0.01-10% of a surfactant selected from anionic, nonionic and amphoteric and cationic surfactants and mixtures thereof.

3. A composition according to 2, wherein the amphoteric surfactant is a betaine.

4. A composition according to any one of claims 1 to 3, wherein the pH of the composition is about 6-8.5.

5. A composition according to any of the preceding claims, wherin the composition has a viscosity of 60 to 3,000 cm²/minute (100 to 5000 centistokes).

## Patentansprüche

1. Stabile, flüssige Waschmittelzusammensetzung mit einem hohen Glyceringehalt, umfassend:
(a) 1 bis 25 Gew.-% an einem oder mehreren Sulfobernsteinsäuremonoestern, die durch die Formeln (I), (II), (III) oder (IV) dargestellt werden; 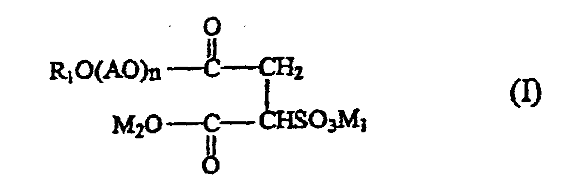 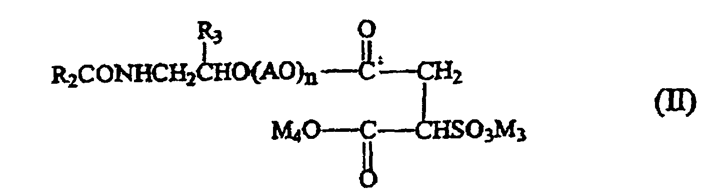 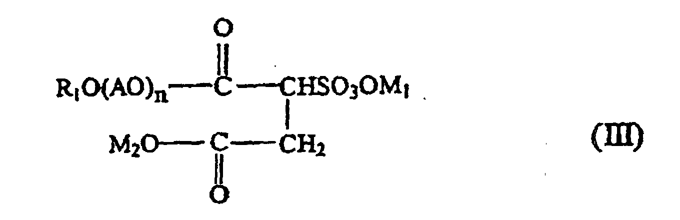 oder 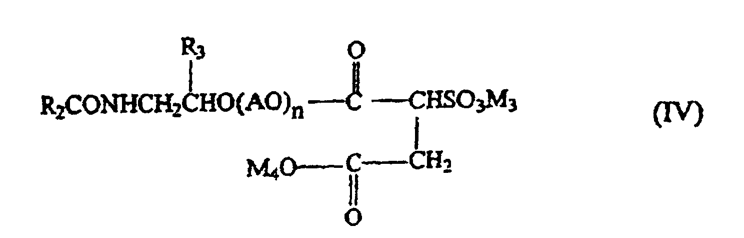 worin jeder von M₁ bis M₄ H, NH₄, ein Alkalimetall oder ein Hydroxyalkylsubstituiertes Ammonium ist. R₁ und R₂ jeweils eine Alkyl- oder Hydroxyalkylgruppe mit etwa 8 bis 20 Kohlenstoffatomen sind, R₃ H oder CH₃ ist, AO eine Oxyalkylengruppe mit 2 oder 3 Kohlenstoffatomen ist und n eine ganze Zahl von 0 bis 20 darstellt;
(b) 10 bis 50 Gew.-% Glycerin;
(c) zumindest 4 Gew.-% einer Alkalimetallseife von C₁₂-C₂₄-Fettsäure und/oder Alkanolseife;
(d) 0,01 bis 5 Gew.-% eines Verdickungsmittels, ausgewählt aus Acrylatcopolymer, Hydroxyalkylcellulose, kationischen Guargummis und Isostearinsäure; und
(e) Rest Wasser.

2. Zusammensetzung nach Anspruch 1, wobei die Zusammensetzung zusätzlich 0,01 bis 10 % eines oberflächenaktiven Mittels umfaßt, ausgewählt aus anionischen, nicht-ionischen und amphoteren und kationischen oberflächenaktiven Mitteln und Gemischen hiervon.

3. Zusammensetzung nach Anspruch 2, wobei das amphotere oberflächenaktive Mittel Betain ist.

4. Zusammensetzung nach einem der Ansprüche 1 bis 3, wobei der pH der Zusammensetzung etwa 6 bis 8,5 beträgt.

5. Zusammensetzung nach einem der vorhergehenden Ansprüche, wobei die Zusammensetzung eine Viskosität von 60 bis 3.000 cm²/Minute (100 bis 5.000 Centistokes) aufweist.

## Revendications

1. Composition détergente liquide stable à teneur élevée en glycérol comprenant :
(a) 1 à 25 % en poids d'un ou plusieurs monoesters d'acide sulfosuccinique représentés par les formules (I), (II), (III) ou (IV) 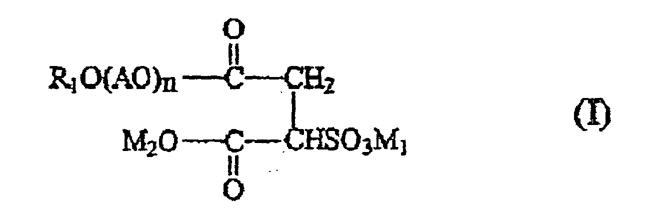 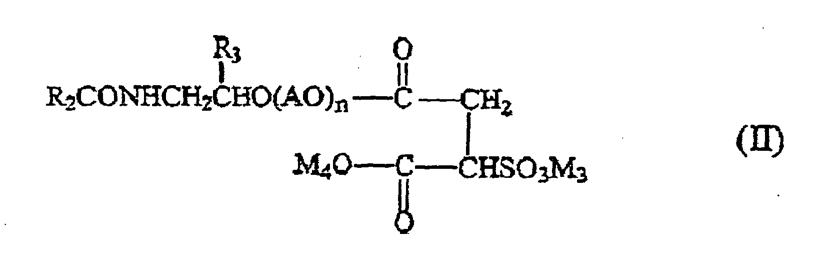 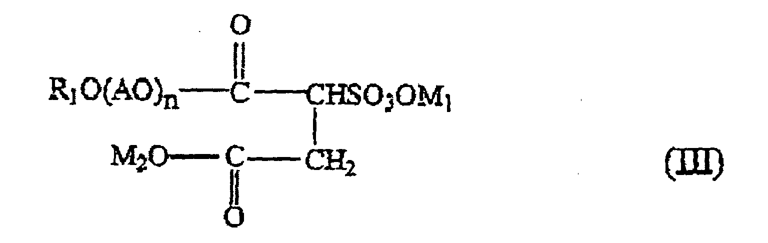 ou 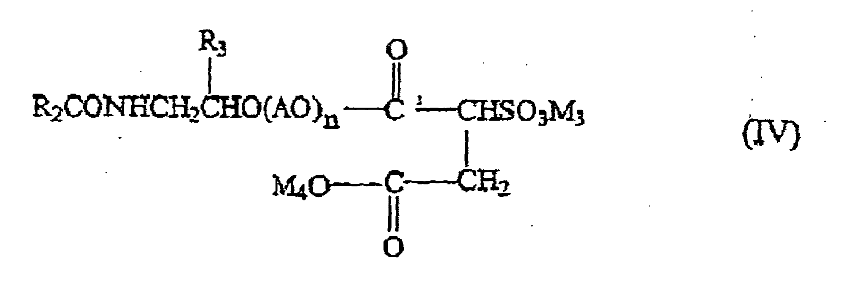 dans lesquelles chacun des M₁ à M₄ est H, NH₄, un métal alcalin ou un ammonium substitué par un hydroxyalkyle ; R₁ et R₂ sont chacun un groupe alkyle ou un groupe hydroxyalkyle ayant 8 à 20 atomes de carbone ; R₃ est H ou CH_{3 ;} AO est un groupe oxyalkylène ayant 2 ou 3 atomes carbone, et n est un entier ayant pour valeur 0 à 20 ;
(b) 10 à 50 % en poids de glycérol ;
(c) au moins 4 % en poids d'un savon de métal alcalin d'acide gras en C₁₂ à C₂₄ et/ou d'un savon d'alcanol ; et
(d) 0,01 à 5 % en poids d'un épaississant choisi parmi un copolymère acrylate, un hydroxy alkyl cellulose; des gommes de guar cationiques et l'acide isostéarique ; et
(e) le reste étant de l'eau

2. Composition selon la revendication 1, dans laquelle la composition comprend en plus 0,01 à 10 % d'un tensioactif choisi parmi les tensioactifs anioniques, non ioniques et amphotères et cationiques et les mélanges de ceux-ci.

3. Composition selon la revendication 2, dans laquelle le tensioactif amphotère est une bétaïne.

4. Composition selon l'une quelconque des revendications 1 à 3, dans laquelle le pH de la composition est environ 6 à 8,5.

5. Composition selon l'une quelconque des revendications précédentes, dans laquelle la composition a une viscosité de 60 à 3 000 cm²/minute (100 à 5 000 centistokes).
